(19) **Europäisches Patentamt / European Patent Office / Office européen des brevets**

(11) **EP 2 692 360 A1**

(12) # EUROPEAN PATENT APPLICATION
published in accordance with Art. 153(4) EPC

(43) Date of publication:
05.02.2014 Bulletin 2014/06

(21) Application number: 12763724.7

(22) Date of filing: 28.03.2012

(51) Int Cl.:
*A61K 47/24* (2006.01)     *A61K 9/107* (2006.01)
*A61K 31/5575* (2006.01)     *A61K 47/12* (2006.01)
*A61K 47/44* (2006.01)

(86) International application number:
PCT/JP2012/058185

(87) International publication number:
WO 2012/133554 (04.10.2012 Gazette 2012/40)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 31.03.2011  JP 2011080876
31.03.2011  JP 2011080877
06.09.2011  JP 2011194203
06.09.2011  JP 2011194204

(71) Applicant: FUJIFILM Corporation
Tokyo 106-0031 (JP)

(72) Inventors:
• TSUJIHATA Shigetomo
Ashigarakami-gun, Kanagawa (JP)
• TANISAKA Hiroki
Ashigarakami-gun, Kanagawa (JP)
• NAGATA Kouzou
Ashigarakami-gun, Kanagawa (JP)
• IZUMI Yasuyuki
Ashigarakami-gun, Kanagawa (JP)

(74) Representative: HOFFMANN EITLE
Patent- und Rechtsanwälte
Arabellastrasse 4
81925 München (DE)

(54) **FAT EMULSION CONTAINING PROSTAGLANDIN**

(57)     Provided are prostaglandin-containing fat emulsion in which the prostaglandin has improved stability and which have excellent emulsion stability, excellent transparency, and a long shelf life.

One is a fat emulsion which has a pH of 4.5 to 6.0 and includes a prostaglandin compound, an oil ingredient, a lecithin, the content of which is 0.15 times or more by mass the content of the oil ingredient, a water-soluble acid having a pKa of 4.0 to 6.0 and having a dissociable group or a salt thereof, and water. Another is a fat emulsion which includes a prostaglandin compound, an oil ingredient, a lecithin, the content of which is 500 to 5,000 times by mass the content of the prostaglandin compound and is 0.5 to 10 times by mass the content of the oil ingredient, and water, and in which the content of a higher fatty acid is 0.06 times or less by mass the content of the lecithin.

**Description**

Technical Field

The present invention relates to prostaglandin-containing fat emulsion capable of being administered through intravenous injection, an injection preparation which includes the prostaglandin-containing fat emulsion, and a process for producing a pre-filled syringe preparation. The invention further relates to a process for producing the injection preparation.

Background Art

Fat emulsions for intravenous injection were developed as one form of prostaglandin E1 ($PGE_1$) preparations, and are on the market under the names of "Liple Injection" (Mitsubishi Tanabe Pharma Corp.), "Palux inj." (Taisho Pharmaceutical Co., Ltd.), etc.

However, since prostaglandin E1 as the active ingredient is susceptible to decomposition, it is necessary that the prostaglandin E1 fat emulsions should be stored in a light-shielded environment of 5°C or lower, and the useful life thereof, as provided by law, is 1 year, which is shorter than those of ordinary preparations. Such preparations cause an increase in the cost of drug management in the stage of distribution and in clinical fields. There is hence an earnest desire for development of a preparation having a long useful life.

Various investigations have been made so far in order to enhance the stability of prostaglandin E1.

For example, it was found that the stability of the prostaglandin is improved by using a purified phospholipid (see patent document 1), by incorporating substantially no higher fatty acid (see patent document 2), etc. However, the methods described in those documents are not considered to bring about a sufficient effect in improving the stability of the prostaglandin, and there are even cases where the fat emulsion comes to have reduced emulsion stability. There has hence been a need for a prolongation of the effective life of the preparations.

In patent document 3, it has been reported that the stability of a prostaglandin improves at a specific emulsifying agent/oil ratio.

Meanwhile, since the fat emulsions containing prostaglandin E1 have a milk-white appearance, it is difficult to detect inclusion of foreign matter which occurs upon ampoule opening, contamination with microorganism, or the presence of coarse particles which generated during storage. Consequently, those fat emulsions are considered to be preparations which are extremely difficult to manage in clinical fields.

The method described in patent document 3 undoubtedly produces the effect of improving the stability of the prostaglandin. However, it was found that since the lecithin which is present in excess is hydrolyzed, the effect of improving long-term storability is insufficient. Meanwhile, citric acid and a specific amino acid are known as stabilizers for fat emulsions (patent document 4). However, although these stabilizers are known to have the effect of inhibiting the discoloration of fat emulsions, it has not been ascertained that the stabilizers have the effect of stabilizing the drug packed in a sealed container. In particular, since the pH has been adjusted to 6.5-7.5, the stabilizers are unable to inhibit, at such a pH, the prostaglandin from decomposing. Furthermore, since citric acid is apt to cause demulsification, the drug must be emulsified under the conditions of a pH exceeding 6.0, from the standpoint of obtaining emulsion stability.

It is, however, known that the stability of the prostaglandin rapidly decreases as the pH increases beyond 6.0. With the method described in patent document 4, it is difficult to obtain a fat emulsion having high storage stability.

Under these circumstances, there is a need for development of a prostaglandin-containing fat emulsion which is excellent in terms of the stability of the active ingredient (prostaglandin), emulsion stability, and transparency.

Prior-Art Documents

Patent Documents

Patent Document 1: JP-B-8-18989
Patent Document 2: JP-A-4-338333
Patent Document 3: International Publication WO 2009/93650
Patent Document 4: JP-A-8-81360

Summary of the Invention

Problems that the Invention is to Solve

Although the method described in patent document 3 undoubtedly produces the effect of improving the stability of a prostaglandin, it was found that since the lecithin which is present in excess is hydrolyzed, the effect of improving long-term storability is insufficient. Meanwhile, it was also found that in the case where the amount of the lecithin added is small, a larger proportion of the prostaglandin is present in a free state in the aqueous phase and, hence, the effect of the drug is not sufficiently obtained.

A first object in the first aspect of the invention is to improve the stability of a prostaglandin and to provide a fat emulsion which has excellent emulsion stability and a long shelf life. A second object is to provide a fat emulsion having high transparency. A third object is to provide an injection preparation and a pre-filled syringe which each include or contain a prostaglandin-containing fat emulsion, and to provide a process for producing an injection preparation which can be easily sterilized.

A first object of the second aspect of the invention is to improve the stability of a prostaglandin and to provide a fat emulsion which has excellent emulsion stability and a long shelf life. A second object is to provide a fat emulsion having high transparency and to provide a fat emulsion which shows a high medicinal effect. A third object is to provide an injection preparation including a prostaglandin-containing fat emulsion and to provide a process for producing an injection preparation which can be easily sterilized. Means for Solving the Problems

The invention includes the following configurations.

[1] A prostaglandin-containing fat emulsion which is a fat emulsion comprising:

a prostaglandin compound,
an oil ingredient,
a lecithin,
a water-soluble acid having a pKa of 4.0 to 6.0 and having a dissociable group or a salt thereof, and
water,
wherein the content of the lecithin is 0.15 times or more by mass the content of the oil ingredient, and
the fat emulsion has a pH of 4.5 to 6.0.

[2] The prostaglandin-containing fat emulsion according to [1],
wherein the water-soluble acid or salt thereof is contained in an amount of 0.01 mmol/L to 5 mmol/L.
[3] The prostaglandin-containing fat emulsion according to [1] or [2],
wherein the water-soluble acid is citric acid.
[4] The prostaglandin-containing fat emulsion according to any one of [1] to [3],
wherein the content of the lecithin in the fat emulsion is 0.3 times or more by mass the content of the oil ingredient.
[5] The prostaglandin-containing fat emulsion according to any one of [1] to [4],
wherein the content of the oil ingredient in the fat emulsion is 0.01 to 5% by mass based on the fat emulsion.
[6] The prostaglandin-containing fat emulsion according to any one of [1] to [5] characterized by further containing a higher fatty acid, the content of the higher fatty acid in the fat emulsion being up to 0.06 times by mass the content of the lecithin.
[7] The prostaglandin-containing fat emulsion according to any one of [1] to [6],
wherein the prostaglandin compound is prostaglandin E1.
[8] A prostaglandin-containing fat emulsion which is a fat emulsion comprising:

a prostaglandin compound,
an oil ingredient,
a lecithin, and
water,
wherein the content of the lecithin is 500 to 5,000 times by mass the content of the prostaglandin compound,
the content of the lecithin is 0.3 to 10 times by mass the content of the oil ingredient, and
the content of a higher fatty acid is 0.06 times by mass or less the content of the lecithin.

[9] The prostaglandin-containing fat emulsion according to [8],
wherein the content of the lecithin is 0.4 to 2% by mass based on the whole fat emulsion.
[10] The prostaglandin-containing fat emulsion according to [8] or [9],
wherein the portion of the prostaglandin compound which is present in a free state in the aqueous phase accounts for 10% or less of the prostaglandin compound in the fat emulsion.

[11] The prostaglandin-containing fat emulsion according to any one of [8] to [10], wherein the content of the oil ingredient is 0.2 to 5% by mass based on the whole fat emulsion.

[12] The prostaglandin-containing fat emulsion according to any one of [8] to [11], which further contains a water-soluble acid having a pKa of 4 to 6 and having a dissociable group or a salt thereof.

[13] The prostaglandin-containing fat emulsion according to [12], wherein the content of the water-soluble acid or salt thereof in the fat emulsion is 0.01 mmol/L to 5 mmol/L.

[14] The prostaglandin-containing fat emulsion according to any one of [8] to [13], wherein the fat emulsion has a pH in the range of 4.5 to 6.0.

[15] The prostaglandin-containing fat emulsion according to any one of [1] to [14], wherein an average particle diameter of the fat emulsion as measured by a light scattering method is 30 to 150 nm.

[16] The prostaglandin-containing fat emulsion according to any one of [1] to [15], wherein the lecithin is yolk lecithin containing phosphatidylcholine in an amount of 98% by mass or more.

[17] The prostaglandin-containing fat emulsion according to any one of [1] to [16], wherein the oil ingredient is soybean oil.

[18] The prostaglandin-containing fat emulsion according to any one of [1] to [17], wherein the prostaglandin-containing fat emulsion is a prostaglandin-containing fat emulsion which has undergone sterilization by filtration.

[19] An injection preparation which comprises the prostaglandin-containing fat emulsion according to any one of [1] to [18].

[20] A pre-filled syringe preparation comprising a syringe packed with the prostaglandin-containing fat emulsion according to any one of [1] to [18] or with the injection preparation according to [19].

[21] A process for producing the injection preparation according to [19] or [20], which contains a step of sterilizing the prostaglandin-containing fat emulsion according to [19] or [20] by filtration.

Effects of the Invention

According to the configurations of the invention, not only the stability of prostaglandin is greatly improved but also the emulsion stability of the fat emulsions is improved. In addition, an unexpected effect that coarse particles are diminished was found out. Namely, according to the configurations of the invention, it is possible to provide a prostaglandin-containing fat emulsion, an injection preparation, and a pre-filled syringe preparation which are capable of administration through intravenous injection and have a greatly improved shelf life as compared with conventional products. Since this fat emulsion has improved transparency, inclusion of foreign matter can be easily detected and this preparation is effective also from the standpoint of drug management in clinical fields. Furthermore, it is possible to provide a prostaglandin-containing fat emulsion which produces a high medicinal effect.

Modes for Carrying Out the Invention

The prostaglandin-containing fat emulsion according to the first aspect of the invention, which can be intravenously administered, is a fat emulsion that includes a prostaglandin compound, an oil ingredient, a lecithin, a water-soluble acid having a pKa of 4.0 to 6.0 and having a dissociable group or a salt of the acid, and water, and is characterized in that the content of the lecithin is 0.15 times or more by mass the content of the oil ingredient, and the fat emulsion has a pH of 4.5 to 6.0.

It has conventionally been widely known that the presence of a fatty acid in a fat emulsion reduces the stability of the prostaglandin contained in the fat emulsion. However, the inventors have found out an unexpected effect that the stability of the prostaglandin contained in a fat emulsion is remarkably improved by causing a specific water-soluble acid to present therein.

The prostaglandin-containing fat emulsion according to the second aspect of the invention is a fat emulsion which includes a prostaglandin compound, an oil ingredient, a lecithin, and water, and in which the content of the lecithin is 500 to 5,000 times by mass the content of the prostaglandin compound, the content of the lecithin is 0.3 to 10 times by mass the content of the oil ingredient, and the content of a higher fatty acid is 0.06 times or less by mass the content of the lecithin.

The prostaglandin-containing fat emulsion according to the second aspect of the invention can be intravenously administered, and the proportions of the prostaglandin compound, oil ingredient, lecithin, and water have been limited to specific ranges. Since the proportions of these components are within specific ranges, it is possible to provide a medicinal preparation which can satisfy the stability of the prostaglandin, emulsion stability, transparency of the fat emulsion, and the effect of the drug.

Here, the expression "times by mass" means what times the amount by mass of the component is.

<Water-soluble Acid>

The fat emulsion according to the second aspect of the invention contains either a water-soluble acid having a pKa of 4.0 to 6.0 and having a dissociable group or a salt thereof.

It is preferred that the fat emulsion according to the second aspect of the invention contains the water-soluble acid.

Here, the acid dissociation constant pKa is a value determined in 25°C water. In the case of a multifunctional acid, this acid may be one in which any of the multiple acid dissociation constants is in the range of 4.0 to 6.0.

This water-soluble acid preferably is an organic acid. More preferred is a carboxylic acid having 2 to 10 carbon atoms. Specific examples of the water-soluble acid include acetic acid (pKa=4.76), butyric acid (pKa=4.63), benzoic acid (pKa=4.00), citric acid (pKa1=3.15, pKa2=4.77, pKa3=6.40), succinic acid (pKa1=4.00, pKa2=5.24), tartaric acid (pKa1=3.2, pKa2=4.8), phthalic acid (pKa1=2.94, pKa2=5.41), fumaric acid (pKa1=2.85, pKa2=4.10), maleic acid (pKa1=1.75, pKa2=5.83), and malic acid (pKa1=3.40, pKa2=5.13). Preferred of these are acetic acid and citric acid. Especially preferred is citric acid. Here, the values of acid dissociation constant pKa are ones determined in 25°C water. In the case of a polyfunctional acid, this acid may be one in which any of the multiple acid dissociation constants is in that range.

In this fat emulsion of the invention, an appropriate amount of sodium hydroxide, hydrochloric acid, phosphoric acid, phosphoric acid salts, citric acid, and citric acid salts may be used in a suitable combination as a pH regulator in order to regulate the pH to that value. In particular, it is preferred to add a citric acid/phosphoric acid buffer or a citric acid buffer as a pH regulator in order to keep the pH during storage at a value within the preferred range.

A plurality of such water-soluble acids may be used in combination.

The water-soluble acid may be contained in the form of a salt, or may constitute a buffer system. The kind of the salt is not particularly limited, and examples thereof include salts with alkali metals or alkaline earth metals. Preferred are salts with sodium, potassium, or calcium.

It is preferred that the water-soluble acid or salt thereof is contained in the fat emulsion in an amount of 0.001 mmol/L to 50 mmol/L. The content thereof is more preferably 0.005 mmol/L to 10 mmol/L, especially preferably 0.01 mmol/L to 5 mmol/L. By regulating the content thereof so as to be within that range, the effect of stabilizing the prostaglandin is sufficiently obtained and emulsion stability can be maintained. That range is therefore preferred.

<Prostaglandin Compound>

Examples of the prostaglandin compound, among the ingredients constituting the fat emulsions of the invention, include prostaglandin E1 (PGE$_1$), prostaglandin A2 (PGA2), prostaglandin D2 (PGD2), prostaglandin E2 (PGE$_2$), prostaglandin F1$\alpha$ (PGF1$\alpha$), prostaglandin I2 (PGI2), and derivatives thereof. Preferred of these in the invention is prostaglandin E1 (PGE$_1$), which is much in demand in fat emulsions. The invention is especially effective with PGE$_1$.

A plurality of prostaglandins may be used in combination.

The prostaglandin-containing fat emulsions of the invention contain a prostaglandin compound. Specifically, the content of the prostaglandin compound in each fat emulsion of the invention is preferably 0.00001 to 0.01 % by mass, more preferably 0.0001 to 0.005% by mass, even more preferably 0.0003 to 0.001% by mass.

In the second aspect of the invention, the proportion of the portion of the prostaglandin compound which is present in a free state in the aqueous phase to the prostaglandin compound contained in the fat emulsion is preferably 10% or less, more preferably 0.1 to 10%, even more preferably 0.1 to 8%, especially preferably 0.1 to 6%. It is known that the prostaglandin compound, which shows a medicinal effect, in a prostaglandin-containing fat emulsion is contained in the fat particles and, hence, this prostaglandin compound is prevented from being deactivated in the lungs and the property of targeting the inflamed region is enhanced. Consequently, by reducing the proportion of the prostaglandin which is present in a free state in the aqueous phase to all prostaglandin compound contained in the fat emulsion, the effect of the drug can be prevented from decreasing. Incidentally, the free prostaglandin in the aqueous phase can be separated by dialysis or ultrafiltration.

<Lecithin>

The fat emulsions of the invention contain a lecithin.

Here, the lecithin is phosphatidylchloline itself or a mixture which contains at least phosphatidylchloline.

The phosphatidylchloline-containing mixture generally is a mixture which can contain phosphatidylserine, phosphatidylethanolamine, phosphatidylinositol, N-acylphosphatidylethanolamines, phosphatidylglycerol, phosphatidic acid, lysophosphatidylcholine, lysophosphatic acid, sphingomyelin, sphingoethanolamine, and the like other than phosphatidylchloline.

The lecithin may be either a synthesized product or one derived from a natural substance. Examples thereof generally include yolk lecithin (lecithin derived from yolk; the same applies hereinafter), soybean lecithin, cottonseed lecithin, rapeseed lecithin, and corn lecithin. Preferred as the lecithin to be used in the invention are yolk lecithin and soybean lecithin. More preferred is yolk lecithin. Purified yolk lecithin obtained by purifying yolk lecithin is preferred, and highly purified yolk lecithin is more preferred. The lecithin in the invention preferably is yolk lecithin which includes phosphatidylcholine and has a phosphatidylcholine content of 96% or higher, and more preferably is yolk lecithin having a phosphatidylcholine content of 98% by mass or higher. This lecithin is suitable for use in fat emulsions to be administered through intravenous injection.

As the yolk lecithin having a phosphatidylcholine content of 98% by mass or higher, use can be made of the products enumerated in *Iyakuhin Tenkabutsu Jiten 2007* (Yakuji Nippo Ltd.) under the name "Highly Purified Yolk Lecithin". Specifically, examples thereof include PC-98N (manufactured by Q. P. Corp.)

The content of the lecithin in the fat emulsion according to the first aspect of the invention is preferably 100 to 20,000 times by mass, more preferably 500 to 10,000 times by mass, especially preferably 1,000 to 5,000 times by mass, the content of the prostaglandin compound. Meanwhile, the content of the lecithin, based on the fat emulsion, is desirably 0.1% by mass or higher, preferably 0.2% by mass or higher, more preferably 0.3% by mass or higher, even more preferably 0.5% by mass or higher, especially preferably 1.2% by mass or higher. The content thereof is preferably 3% by mass or less, more preferably 2% by mass or less. When the content of the lecithin is within that range, the emulsion stability is high and the amount of the prostaglandin present in a free state in the water of the fat emulsion is small, thereby producing a high medicinal effect. That lecithin content range is hence preferred.

The fat emulsion according to the second aspect of the invention is characterized in that the content of the lecithin is 500 to 5,000 times by mass the content of the prostaglandin compound. The content of the lecithin, relative to the content of the prostaglandin compound, is preferably 1,000 to 5,000 times by mass, especially preferably 2,000 to 4,500 times by mass.

Furthermore, the content of the lecithin in the fat emulsion of the invention is preferably 0.4 to 2% by mass, more preferably 0.5 to 1.9% by mass, especially preferably 0.6 to 1.8% by mass. When the content of the lecithin is within that range, the emulsion stability can be improved and the proportion of the prostaglandin compound which is present in a free state in the water to all prostaglandin in the fat emulsion can be reduced, thereby producing a high medicinal effect. That lecithin content range is hence preferred.

<Oil Ingredient>

The fat emulsions of the invention contain an oil ingredient.

As the oil ingredient to be used in the invention, it is preferred to use fatty acid glyceride (monoglyceride, diglyceride, triglyceride, and a mixture of two or more thereof).

As the fatty acid glyceride, use can be made of either medium-chain fatty acid glyceride or long-chain fatty acid glyceride.

The medium-chain fatty acid glyceride is a condensate of a fatty acid having 6 to 12 carbon atoms with glycerin, and examples thereof include TCG-M (Kokyu Alcohol Kogyo), Crodamol GTCC (Croda Japan), Coconard MK (Kao), Coconard RK (Kao), Sunfat MCT-7 (Taiyo Kagaku), Deriosu (Cognis Japan), Panasate (Nippon Oil & Fats), Miglyol 810 (Mitsuba Trading), Miglyol 812 (Mitsuba Trading), Myritol 318 (Cognis Japan), and Panasate 810 (Yuka Sangyo).

The long-chain fatty acid glyceride is a condensate of a fatty acid having 14 or more carbon atoms with glycerin, and examples thereof include soybean oil, olive oil, sesame oil, rapeseed oil, peanut oil, sunflower oil, corn oil, safflower oil, and cottonseed oil. Preferred of these are soybean oil, olive oil, and sesame oil. Especially preferred is soybean oil.

These fatty acid glycerides may be used after further purified by steam distillation or the like.

The content of the oil ingredient in the fat emulsion according to the first aspect of the invention is preferably 0.01 to 10% by mass, more preferably 0.01 to 5% by mass, especially preferably 0.01 to 2% by mass, based on the fat emulsion from the standpoint of making it easy to maintain emulsion stability and transparency while preventing the lecithin from hydrolyzing. Meanwhile, from the standpoint of further diminishing the coarse particles which generate to such a degree that the fat emulsion can be administered through intravenous injection, the content of the oil ingredient is preferably 0.01 to 10% by mass, more preferably 0.01 to 5% by mass, especially preferably 2 to 5% by mass, based on the fat emulsion.

The content of the oil ingredient in the fat emulsion according to the second aspect of the invention is desirably 0.04 to 5% by mass, preferably 0.1 to 5% by mass, more preferably 0.2 to 5% by mass, even more preferably 0.2 to 3% by mass, especially preferably 0.2 to 2% by mass, based on the fat emulsion from the standpoint of making it easy to maintain emulsion stability and transparency while preventing the lecithin from hydrolyzing. Meanwhile, from the standpoint of further diminishing the coarse particles which generate to such a degree that the fat emulsion can be administered through intravenous injection, the content of the oil ingredient is preferably 0.04 to 10% by mass, more preferably 0.2 to 7% by mass, even more preferably 0.2 to 5% by mass, especially preferably 2 to 5% by mass, based on the fat emulsion.

<Mass Ratio of Lecithin to Oil Ingredient>

In the fat emulsion according to the first aspect of the invention, the content of the lecithin is preferably 0.15 to 50 times by mass, more preferably 0.5 to 20 times by mass, especially preferably 0.7 to 10 times by mass, most preferably 0.7 to 6 times by mass, the content of the oil ingredient, from the standpoints of inhibiting the particle diameter from changing with the lapse of time and of attaining emulsion stability. Meanwhile, from the standpoint of further diminishing the coarse particles which generate to such a degree that the fat emulsion can be administered through intravenous injection, the content of the lecithin is preferably 0.15 to 50 times by mass, more preferably 0.2 to 10 times by mass, especially preferably 0.3 to 1 time by mass, most preferably 0.3 to 0.7 times by mass, the content of the oil ingredient.

The fat emulsion according to the second aspect of the invention is characterized in that the content of the lecithin is 0.3 to 10 times by mass the content of the oil ingredient. The mass ratio of the lecithin to the oil ingredient is more preferably 0.7 to 8 times by mass, especially preferably 1 to 5 times by mass. When the content of the lecithin is within this range, the lecithin can be inhibited from hydrolyzing and the fat emulsion has high emulsion stability and is reduced in the amount of the prostaglandin which is present in a free state in the water, thereby producing a high medicinal effect. That range of lecithin content is hence preferred. Meanwhile, from the standpoint of further diminishing the coarse particles which generate to such a degree that the fat emulsion can be administered through intravenous injection, the content of the oil ingredient is preferably 0.3 to 10% by mass, more preferably 0.3 to 1% by mass, especially preferably 0.3 to 0.7% by mass, based on the fat emulsion.

<Higher Fatty Acid>

A higher fatty acid may be incorporated into the fat emulsions of the invention for the purpose of improving the emulsion stability.

The higher fatty acid is a fatty acid having 10 or more carbon atoms, and may be either a saturated fatty acid or an unsaturated fatty acid. In the invention, the higher fatty acid functions as an emulsification aid to improve the emulsion stability of the fat emulsions. Examples of the higher fatty acid to be used in the invention include oleic acid, palmitic acid, stearic acid, linoleic acid, and linolenic acid. Especially preferred is oleic acid.

It is preferred that the fat emulsion according to the first aspect of the invention should contain a higher fatty acid and that the content of the higher fatty acid is 0.06 times or less by mass the content of the lecithin. The content of the higher fatty acid is preferably 0.0001 to 0.06 times by mass, more preferably 0.0001 to 0.03 times by mass, especially preferably 0.0001 to 0.01 times by mass, the content of the lecithin. It is most preferred that substantially no higher fatty acid is added.

In the fat emulsion according to the second aspect of the invention, a higher fatty acid may be incorporated for the purpose of improving the emulsion stability. However, the content thereof is 0.06 times or less by mass, preferably 0.0001 to 0.06 times by mass, the content of the lecithin. In case where the mass ratio of the higher fatty acid exceeds 0.06, the stability of the prostaglandin decreases although the emulsion stability improves. Namely, it is impossible to obtain a prolongation of shelf life.

The mass ratio of the higher fatty acid to the lecithin is more preferably 0.0001 to 0.03, especially preferably 0.0001 to 0.01, from the standpoint of inhibiting the prostaglandin from decomposing. It is most preferred that substantially no higher fatty acid is added.

The expression "substantially no higher fatty acid is added" herein means that any higher fatty acid is not purposely incorporated. For example, a free higher fatty acid yielded by decomposition of an oil ingredient or phospholipid and a free higher fatty acid which came in accidentally and is contained are excluded.

<Emulsifying Agent and Dispersant>

In the fat emulsions of the invention, an emulsifying agent or a dispersant may be further added for the purpose of improving the emulsion stability.

Examples of the emulsifying agent include Poloxamer (polyoxyethylene/polyoxypropylene copolymer), polyoxyl 35 castor oil, polyoxyl 40 hydrogenated castor oil, polyoxyl 60 hydrogenated castor oil, polyoxyethylene sorbitan monolaurate, polyoxyethylene sorbitan monopalmitate, polyoxyethylene sorbitan monostearate, polyoxyethylene sorbitan tristearate, polyoxyethylene sorbitan monooleate, 12-hydroxystearic acid/polyoxyethylene esters, d-$\alpha$-tocopheryl poly-ethylene glycol succinate, sorbitan/fatty acid esters, and sorbitan sesquioleate.

Examples of the dispersant include human serum albumin, purified gelatin, polyvinylpyrrolidone, ursodesoxycholic acid, ursodesoxycholic acid salts, desoxycholic acid, and desoxycholic acid salts.

The amount of these emulsifying agents and dispersants to be added is not particularly limited so long as these additives do not affect the stability of the fat emulsions. However, in the case of adding an emulsifying agent or a dispersant, the amount thereof is generally 0.1 time or more by mass the amount of the oil ingredient, and the amount thereof is preferably 20 times by mass or less, more preferably 10 times by mass or less, even more preferably 5 times by mass or less.

<Other Ingredients>

The fat emulsions of the invention may contain an isotonicity agent (e.g., glycerin, glucose, or sodium chloride), an antioxidant (e.g., ascorbic acid and salts thereof, dibutylhydroxyanisole, dibutylhydroxytoluene, $\alpha$-tocopherol, or D-sorbitol), and a pH regulator (sodium hydroxide, hydrochloric acid, or phosphoric acid) according to need.

<Particle Diameter of Fat Emulsion>

The average particle diameter of each fat emulsion of the invention (immediately after emulsification), as determined by a dynamic light scattering method, is preferably 30 to 150 nm, more preferably 30 to 120 nm, especially preferably 30 to 100 nm. By regulating the fat emulsion so as to have a particle diameter within that range, the transparency of the fat emulsion is improved and, hence, it becomes easy to find out inclusion of foreign matter or contamination with microorganisms. Consequently, in clinical fields, any preparation in which a problem concerning the use thereof has arisen can be easily found out. Furthermore, since this fat emulsion can be sterilized by filtration, that particle diameter range is preferred also from the standpoint of diminishing decomposition products. In the case where the fat emulsion of the invention is sterilized by filtration, this fat emulsion can pass through the filter medium for sterilization by filtration, without clogging the filter medium, so long as the particle diameter of the fat emulsion is within that range.
That particle diameter can be attained by using any of the emulsifiers which will be described later and by controlling the treatment pressure and the number of treatments.
The particle diameter of a fat emulsion can be measured with a commercial particle size distribution analyzer or the like. Known as methods for analyzing particle size distribution are light microscopy, confocal laser microscopy, electron microscopy, atomic force microscopy, static light scattering method, laser diffractometry, dynamic light scattering method, centrifugation, electric pulse counting method, chromatography, ultrasonic  attenuation method, and the like. Devices according to the respective principles are on the market.
It is preferred that a light scattering method, more preferably a dynamic light scattering method, or laser diffractometry should be used for the particle diameter measurement in the invention from the standpoints of particle diameter range and ease of measurement. Examples of commercial measuring apparatus employing dynamic light scattering include Nanotrac UPA (Nikkiso Co., Ltd.), dynamic light-scattering type particle size distribution analyzer LB-550 (Horiba Ltd.), and particle size analyzer FPAR-1000 for thick systems (Otsuka Electronics Co., Ltd.).
In particular, the particle diameter of the fat emulsion in the invention is a value measured with FPAR-1000. Specifically, the median diameter of a scattered intensity distribution obtained by the CONTIN method was taken as the particle diameter.

<pH of the Fat Emulsion>

The pH of each fat emulsion of the invention is preferably in the range of 4.5 to 6.0, more preferably in the range of 4.8 to 5.8, especially preferably in the range of 5.0 to 5.5. By regulating the pH thereof so as to be within this range, the stability of the prostaglandin can be further enhanced. In case where the pH thereof is less than 4.5, not only the prostaglandin has reduced stability but also there are cases where this fat emulsion has reduced emulsion stability. On the other hand, in case where the pH thereof exceeds 6.0, the stability of the prostaglandin decreases as the pH increases.
Incidentally, the values of hydrogen ion exponent pH were measured at 25°C.

<Process for Producing Fat Emulsion>

A fat emulsion of the invention can be produced, for example, by adding water according to need to a mixture of a prostaglandin compound, an oil ingredient, and a lecithin. Although methods for producing the fat emulsion of the invention are not particularly limited, an especially preferred method is to use two or more kinds of emulsifiers in combination in such a manner, for example, that the mixture is emulsified using an ordinary emulsifier in which shearing is utilized, such as a stirrer, impeller, homomixer, or continuous flow type shearing device, and the resultant emulsion is passed through a high-pressure homogenizer. By using a high-pressure homogenizer, the emulsified particles can be converted to more even fine droplets.
Examples of the high-pressure homogenizer include a chamber type high-pressure homogenizer which has a chamber having a fixed channel for liquids to be treated and a homogenizing valve type high-pressure homogenizer which has a homogenizing valve. Of these homogenizers, the homogenizing valve type high-pressure homogenizer is in extensive use in the field of emulsification for producing, in particular, foods, cosmetics, and the like because the width of the channel for liquids to be treated can be easily regulated and, hence, the pressure and flow rate during operation can be set at will in wide ranges. In contrast, the chamber type high-pressure homogenizer is used in applications where an ultrahigh pressure is required, because a mechanism for elevating pressure is easy to construct therein although the degree of freedom of operation is low.

Examples of the chamber type high-pressure homogenizer include Microfluidizer (Microfluidics Corp.), Nanomizer (Yoshida Kikai Co., Ltd.), and Ultimizer (Sugino Machine Ltd.).

Examples of the homogenizing valve type high-pressure homogenizer include Gaulin type homogenizers (PVA Inc.), Rannie type homogenizers (Rannie Inc.), high-pressure homogenizers (Niro Soavi S.p.A.), homogenizers (Sanwa Engineering Ltd.), high-pressure homogenizers (Izumi Food Machinery Co., Ltd.), and ultrahigh-pressure homogenizers (Ika Company).

A dispersion process with a high-pressure homogenizer is thought to be attributable to the high shearing force which generates when the liquid passes through an exceedingly narrow (small) gap at a high speed. The magnitude of this shearing force is approximately proportional to the pressure. Namely, the higher the pressure, the higher the shearing force, i.e., dispersing force, that is applied to the particles dispersed in the liquid. However, since the kinetic energy of a liquid which is flowing at a high speed is mostly converted to heat, the temperature of the liquid rises as the pressure increases. There are hence cases where the deterioration of components of the dispersion liquid and re-agglomeration of the particles are accelerated due to the elevated temperature. Consequently, although a high-pressure homogenizer has an optimal point of pressure, this optimal point is thought to vary depending on the substance to be dispersed and also on the target particle diameter. In the invention, it is preferred to conduct the treatment at a homogenizer pressure of preferably 50 MPa or higher, more preferably 50 to 250 MPa, even more preferably 100 to 250 MPa. By conducting the dispersion process under high-pressure conditions within that range, the emulsion can be regulated so as to have the particle diameter. That pressure range is hence preferred. It is preferred that the emulsion should be cooled by passing the emulsion through any cooler within 30 seconds, preferably within 3 seconds, after passing through the chamber.

Another effective method for obtaining fine emulsified particles is to use an ultrasonic homogenizer. Specifically, a method which has been known is to emulsify the mixture using an ordinary emulsifier in which shearing is utilized, such as those described above, and then propagate an ultrasonic wave to the emulsion at a frequency of 15 to 40 kHz. Examples of high-power ultrasonic homogenizers include ultrasonic homogenizers US-1200T, RUS-1200T, and MUS-1200T (all manufactured by Nihonseiki Kaisha Ltd.) and ultrasonic processors UIP2000, UIP-4000, UIP-8000, and UIP-16000 (all manufactured by Hielscher GmbH). By using any of these high-power ultrasonic propagators under the conditions of a frequency of 25 kHz or less, preferably 15 to 20 kHz, and an energy density in the dispersing part of 100 W/cm$^2$ or higher, preferably 120 W/cm$^2$, the mixture can be finely emulsified.

An ultrasonic homogenizer may be used in combination with the ultrahigh-pressure homogenizer described above. Namely, by emulsifying the mixture using an ordinary emulsifier in which shearing is utilized and then subjecting the resultant emulsion to a dispersion treatment with the ultrahigh-pressure homogenizer, the efficiency of dispersing with the ultrahigh-pressure homogenizer is heightened and the number of passes can be reduced. In addition, coarse particles are diminished, making it possible to obtain a high-quality emulsion. Furthermore, by further conducting ultrasonic propagation after the emulsification with an ultrahigh-pressure homogenizer, coarse particles can be diminished. It is also possible to repeatedly conduct these steps in any desired order; for example, a dispersion process at an ultrahigh pressure and ultrasonic propagation are alternately performed.

<Forms of Preparation>

The present invention relates also to an injection preparation which includes a prostaglandin-containing fat emulsion.

The form of this preparation including a fat emulsion is not limited so long as the preparation is suitable for use as an injection preparation. Specific examples thereof include preparations packed into containers, such as an ampoule, vial, pre-filled syringe, and bag.

The capacity, material, and shape of such a container can be suitably selected from the standpoints of the amount of the prostaglandin-containing fat emulsion to be packed and ease of use. It is preferred to diminish the gas, which occupies the space, or to conduct nitrogen displacement during the filling, because the stability is further improved thereby.

Furthermore, it is preferred that the inner surfaces of those containers have been treated by silicoating or the like.

Among those preparations, an ampoule preparation or a pre-filled syringe preparation is preferred. More preferred is pre-filled syringe preparation.

The present invention relates also to a pre-filled syringe preparation in which the syringe has been packed with a prostaglandin-containing fat emulsion or injection preparation.

A pre-filled syringe preparation is a syringe in which a preparation having the use-time concentration has been packed in a use amount in order to avoid mistakes in dilution during preparation or the mistake of taking a wrong drug or to prevent bacterial contamination, a decrease in activity, etc. due to installment use or due to storage. Such a pre-filled syringe preparation is preferred also from the standpoints of reducing the risk of infection and improving the productivity of the labor of medical personnel, etc.

The syringe to be used for the injection preparation and for the pre-filled syringe preparation is not particularly limited, and a conventionally known syringe can be suitable.

The syringe according to the invention can be configured of a syringe barrel, a gasket, etc. It is preferred that the syringe barrel is a cylindrical body in which the opening at one end (base-end-side opening) has been fitted with the gasket and which has, at the other end (fore-end opening), a discharge port through which the prostaglandin-containing fat emulsion is discharged by pushing the gasket. Before use, the discharge port is usually fitted with a cap so that the medicinal preparation can be held with the cap and the gasket. A plunger rod may have been linked to the gasket. The distance from the base end of the syringe barrel to the end of the cap and the inner diameter of the syringe barrel can be suitably determined in accordance with the volume of the medicinal preparation (prostaglandin-containing fat emulsion) to be packed.

Examples of the materials of the syringe barrel and plunger rod to be used in the invention include ordinary plastics or glasses. Examples of the plastics include polyolefins (e.g., polyethylene and polypropylene) and cyclic polyolefins. The capacity can be suitably determined in accordance with the amount of this preparation to be clinically used. Specific examples thereof include one having a capacity of 1-20 mL.

According to need, a syringe barrel made of a glass or plastic can be treated with a silicone or the like through baking or coating fluid application, thereby reducing the sliding resistance of the gasket to facilitate gasket movements within the syringe barrel. It is preferred that the fore-end opening where the discharge port has been formed should have a lure tip shape corresponding to the shape of the instrument to be connected thereto, from the standpoint of ease of connection to a syringe needle or vascular catheter.

The cap to be used in the invention is not particularly limited. However, a cap made of an elastomer, such as a rubber or a thermoplastic elastomer, is preferred. The gasket may have been preferably provided with a means, e.g., a screw part, for connecting the plunger rod thereto. The plunger rod and the gasket may have been molded so as to be integrated with each other.

When this preparation is administered, the fat emulsion of the invention may be used, as such, as an injection preparation and subjected to an intravenous injection, or may be administered by dripping after suitably diluted with a transfusion, e.g., physiological saline.

Examples of methods for sterilizing the fat emulsion include steam sterilization under high pressure and sterilization by filtration. From the standpoints of inhibiting the fat emulsion from suffering drug decomposition or demulsification during sterilization, it is preferred that the fat emulsion is sterilized by filtration. It is also preferred that prior to preparation of the fat emulsions, the liquid ingredients themselves are sterilized by filtration or the liquid ingredients and/or a solution of the solid ingredient(s) are sterilized by filtration.

The present invention relates also to a process for producing the injection preparation, the process including the step of sterilizing the prostaglandin-containing fat emulsion by filtration.

Usually, the sterilization by filtration can be conducted after the prostaglandin-containing fat emulsion has been prepared.

Preferred for use in the sterilization by filtration is a filter medium having a pore diameter of 0.01 to 0.22 $\mu$m. More preferred is a filter medium for sterilization by filtration which has a pore diameter of 0.1 to 0.22 $\mu$m. Commercial filter media can be used for the sterilization by filtration. Specific examples of the filter medium for sterilization by filtration include Sartopore 2 and Sartobran (Sartorius Stedim Japan), Durapore (Millipore Japan), Fluorodyne II, Supor, Fluorodyne EX, Ultipor N66, and Posidyne (Pall Japan).

When sterilization by filtration is conducted, a differential pressure can be applied using a pressure filter, and the differential pressure is preferably 0.01 MPa to 1 MPa, more preferably 0.05MPa to 0.3 MPa. The term differential pressure herein means the difference in pressure between the upstream side (inlet side) and the downstream side (outlet side) in the filtration. Usually, the pressure on the downstream side is atmospheric pressure.

Those conditions can be suitably selected in accordance with the concentrations of the prostaglandin compound, oil ingredient, and lecithin used, the kinds and concentrations of additives which can be contained, etc.

Examples

The invention is explained below in more detail by reference to Examples, but the invention should not be construed as being limited to the following Examples.

In this description, "%" means "% by mass" unless otherwise indicated.

(Example 1-1)

Prostaglandin E1 (Alprostadil, manufactured by Daiichi Fine Chemical Co., Ltd.) was dissolved in ethanol in a concentration of 10 mg/mL. A 42-$\mu$L portion thereof (420 $\mu$g in terms of prostaglandin E1) was mixed with 0.252 g of soybean oil (manufactured by Kaneda Co., Ltd.) and 0.504 g of highly purified yolk lecithin PC-98N (manufactured by Q. P. Corp.). A 2.5% by mass aqueous solution of glycerin separately obtained by mixing concentrated glycerin according to the Japanese Pharmacopoeia (manufactured by Kao Corp.) with purified water was added to that mixture in such an amount as to result in a total amount of 60 mL, and the resultant mixture was stirred. This mixture was treated with a homomixer (15,000 rpm, 12 min) to roughly disperse the ingredients and further treated with a chamber type high-pressure homogenizer to emulsify the mixture. A citric acid/sodium citrate buffer was added to the emulsion so as to result in a final concentration of 0.5 mM to adjust the pH of the emulsion to 5.0. Thus, dispersion liquid 1-1 was produced.

(Examples 1-2 to 1-11, Comparative Examples 1-1, 1-2, and 1-4, and Reference Examples 1-3)

In accordance with the formulations shown in Table 1-1, dispersion liquids 1-2 to 1-15 were produced in the same manner as in Example 1-1. Incidentally, the amounts of hydrochloric acid in Comparative Examples 1-1, 1-2, and 1-4 and Reference Example 1-3 are the amounts of the acid which was added so as to result in the pH values shown in the table.

(Comparative Example 1-4; corresponding to existent medicine)

The same procedure as in Example 1-1 was conducted, except that dispersion liquid 1-16 was produced in accordance with the formulation shown in Table 1-1. Incidentally, the oleic acid was used in such a manner that the given amount of the acid was dissolved in the soybean oil beforehand and emulsified and the pH was thereafter adjusted to 5.3 with sodium hydroxide.

(Evaluation of the Fat Emulsion)

<Particle Diameter Measurement>

The dispersion liquids (fat emulsions) prepared in the Examples and Comparative Examples shown in Table 1-1 were each diluted 10 to 100 times with purified water immediately after the emulsification. The median diameter of a scattered intensity distribution obtained by the CONTIN method with a light-scattering particle size distribution analyzer (FPAR-1000, manufactured by Otsuka Electronics Co., Ltd.) was recorded as the particle diameter.

<pH>

The dispersion liquids prepared in the Examples and Comparative Examples shown in Table 1-1 were each examined, in the undiluted state, for pH with a compact pH meter (manufactured by HORIBA). The measured value was recorded as the pH.

<Particle Diameter Change>

A 2-mL portion was taken out from each of dispersion liquids 1-1 to 1-11 and 1-13 to 1-16 and introduced into a silicoated vial (CS-10, manufactured by Fuji Glass Co., Ltd.), and this vial was fitted with a rubber plug and sealed with aluminum. These dispersion liquids were stored at 40°C for 14 days and then subjected to the particle diameter measurement described above. The resultant change in particle diameter from that measured immediately after the preparation was evaluated according to the following criteria, and the results thereof are shown in Table 1-1.

A: No increase in particle diameter was observed.
B: The increase in particle diameter is 10 nm or less.
C: The increase in particle diameter is larger than 10 nm but less than 20 nm.
D: The increase in particle diameter is 20 nm or larger.

[Table 1]

Table 1-1

| | | Content in fat emulsion, % | | Component compositional ratio | | Acid | | Initial properties | | Evaluation over lapse of time |
|---|---|---|---|---|---|---|---|---|---|---|
| | | A) Lecithin | B) Oil ingredient | Lecithin/ PGE$_1$ (by mass) | Lecithin/ oil (by mass) | | Amount mM | pH | Particle diameter nm | Change in particle diameter 40°C, 14 days |
| | | PC-98N | Soybean oil | | | | | | | |
| Example 1-1 | dispersion liquid 1-1 | 0.84 | 0.42 | 1200 | 2 | citric acid | 0.5 | 5.0 | 63 | A |
| Example 1-2 | dispersion liquid 1-2 | 0.84 | 0.84 | 1200 | 1 | citric acid | 0.5 | 5.0 | 114 | A |
| Example 1-3 | dispersion liquid 1-3 | 0.84 | 1.68 | 1200 | 0.5 | citric acid | 0.5 | 5.0 | 117 | A |
| Example 1-4 | dispersion liquid 1-4 | 0.18 | 0.1 | 257 | 1.8 | citric acid | 0.5 | 5.0 | 54 | B |
| Example 1-5 | dispersion liquid 1-5 | 1.8 | 0.1 | 2571 | 18 | citric acid | 0.5 | 5.0 | 47 | C |
| Example 1-6 | dispersion liquid 1-6 | 1.8 | 0.45 | 2571 | 4 | citric acid | 0.5 | 5.0 | 99 | A |
| Example 1-7 | dispersion liquid 1-7 | 1.8 | 1.8 | 2571 | 1 | citric acid | 0.5 | 5.0 | 93 | A |
| Example 1-8 | dispersion liquid 1-8 | 0.84 | 0.42 | 1200 | 2 | citric acid | 0.25 | 5.0 | 99 | A |
| Example 1-9 | dispersion liquid 1-9 | 0.84 | 0.42 | 1200 | 2 | citric acid | 2 | 5.0 | 102 | A |
| Example 1-10 | dispersion liquid 1-10 | 1.8 | 3.6 | 2903 | 0.5 | citric acid | 1.5 | 5.3 | 89 | A |
| Example 1-11 | dispersion liquid 1-11 | 1.8 | 4.0 | 2903 | 0.45 | citric acid | 1.5 | 5.3 | 93 | A |

(continued)

| | | Content in fat emulsion, % | | Component compositional ratio | | Acid | | Initial properties | | Evaluation over lapse of time |
|---|---|---|---|---|---|---|---|---|---|---|
| | | A) Lecithin | B) Oil ingredient | Lecithin/ PGE$_1$ (by mass) | Lecithin/oil (by mass) | | Amount mM | pH | Particle diameter nm | Change in particle diameter 40°C, 14 days |
| | | PC-98N | Soybean oil | | | | | | | |
| Comparative Example 1-1 | dispersion liquid 1-13 | 0.18 | 0.1 | 257 | 1.8 | hydrochloric acid | appropriate amount | 5.0 | 53 | D |
| Comparative Example 1-2 | dispersion liquid 1-14 | 1.8 | 0.1 | 2571 | 18 | hydrochloric acid | appropriate amount | 5.0 | 49 | D |
| Reference Example 1-3 | dispersion liquid 1-15 | 0.84 | 0.42 | 1200 | 2 | lactic acid | 0.5 | 5.0 | 96 | D |
| Comparative Example 1-4 | dispersion liquid 1-26 | 1.8 | 10 | 2571 | 0.2 | oleic acid | 8.5 | 5.3 | 225 | A |

(Examples 1-12 to 1-20 and Comparative Examples 1-5 to 1-7)

<Evaluation of PGE$_1$ Retention>

A 2-mL portion was taken out from each of dispersion liquids 1-1 to 1-7, 1-10, and 1-11 and comparative dispersion liquids 1-13, 1-14, and 1-16 and introduced into a silicoated vial (CS-10, manufactured by Fuji Glass Co., Ltd.), and this vial was fitted with a rubber plug and sealed with aluminum. These dispersion liquids were stored at 40°C for 7 days or for 14 days and then examined for the amount of prostaglandin E1 by high-performance liquid chromatography. In this examination for quantitative analysis, 1-naphthol was used as an internal reference. The PGE$_1$ retention (%) was calculated using the following equation. The results thereof are shown in Table 1-2.

$$\text{PGE}_1 \text{ retention (\%)} = [(\text{PGE}_1 \text{ concentration after lapse of time})/(\text{initial PGE}_1 \text{ concentration})] \times 100$$

[Table 2]

Table 1-2

| | | PGE$_1$ retention, % | |
|---|---|---|---|
| | | 40°C, 7 days | 40°C, 14 days |
| Example 1-12 | dispersion liquid 1-1 | 92% | 86% |
| Example 1-13 | dispersion liquid 1-2 | 92% | 85% |
| Example 1-14 | dispersion liquid 1-3 | 93% | 85% |
| Example 1-15 | dispersion liquid 1-4 | 88% | 80% |
| Example 1-16 | dispersion liquid 1-5 | 92% | 85% |
| Example 1-17 | dispersion liquid 1-6 | 93% | 86% |
| Example 1-18 | dispersion liquid 1-7 | 94% | 87% |
| Example 1-19 | dispersion liquid 1-10 | 92% | 88% |
| Example 1-20 | dispersion liquid 1-11 | 93% | 87% |
| Comparative Example 1-5 | dispersion liquid 1-13 | 90% | 62% |
| Comparative Example 1-6 | dispersion liquid 1-14 | 83% | 44% |
| Comparative Example 1-7 | dispersion liquid 1-16 | 74% | 56% |

As the Examples in Table 1-1 show, dispersion liquids 1-1 to 1-11 and 1-16, to which citric acid had been added, underwent an increase in particle diameter within an acceptable range or no increase in particle diameter with the lapse of time, and these  dispersion liquids were found to be stable emulsions. On the other hand, in dispersion liquids 1-12 to 1-15, in which an acid having a low pKa had been used (the pKa of lactic acid at 25°C is 3.79), an increase in particle diameter was observed and long-term stability was not obtained.

Furthermore, as the Examples in Table 1-2 show, it was found that dispersion liquids 1-1 to 1-7, 1-10, and 1-11, to which citric acid had been added, produce the unexpected effect of improving the stability of the prostaglandin itself. From a comparison with dispersion liquid 1-16, which corresponds to an existent medicine, it can be presumed that those dispersion liquids of Examples have a shelf life which is at least about 3 times the shelf life of the existent medicine. On the other hand, dispersion liquids 1-13 and 1-14, in which hydrochloric acid, which has a low pKa, was used, had a higher prostaglandin retention than dispersion liquid 1-16, which corresponds to an existent medicine, in the initial stage of the storage but were found to show a considerable decrease in prostaglandin retention after 14 days.

Furthermore, as shown in Table 1-1, dispersion liquids 1-1 to 1-11 according to the present invention each has a particle diameter of 150 nm or less and appear to be in a translucent state. Consequently, the prostaglandin-containing fat emulsion of the present invention not only has a prolonged shelf life but also is expected to facilitate drug management concerning inclusion of foreign matter, etc., because of their high transparency.

<Visual Evaluation of Diluted Liquids for Coarse Particles>

Dispersion liquids 1-1, 1-7, 1-10, and 1-11 were each sampled in an appropriate amount and placed in a 5-mL vial (colorless and transparent), and were diluted 1 to 6 times with purified water. Each vial was placed between a fluorescent lamp and the naked eye located at a distance of 1 m or more from the lamp, and the diluted liquid was visually examined from the lateral side of the vial. In this examination, the diluted liquid was visually evaluated for coarse particles in accordance with the following criteria with respect to the degree in which visually recognizable particles were observed in the liquid. The results thereof are shown in Table 1-3.

D: A sediment is clearly seen.
C: Fine particles are seen in small amount.
B: Fine particles are seen only slightly.
A: There are no visually recognizable particles.

[Table 3]

Table 1-3

| | | Content in fat emulsion, % | | Component compositional ratio | Acid | | Initial properties | | |
|---|---|---|---|---|---|---|---|---|---|
| | | A) Lecithin | B) Oil ingredient | Lecithin/oil (ratio by mass) | | Amount mM | pH | Particle diameter nm | Results of visual evaluation for coarse particles |
| | | PC-98N | Soybean oil | | | | | | |
| Example 1-21 | dispersion liquid 1-1 | 0.84 | 0.42 | 2.0 | citric acid | 0.5 | 5.0 | 63 | C |
| Example 1-22 | dispersion liquid 1-7 | 1.8 | 1.8 | 1.0 | citric acid | 0.5 | 5.0 | 93 | B |
| Example 1-23 | dispersion liquid 1-10 | 1.8 | 3.6 | 0.5 | citric acid | 1.5 | 5.3 | 89 | A |
| Example 1-24 | dispersion liquid 1-11 | 1.8 | 4.0 | 0.45 | citric acid | 1.5 | 5.3 | 93 | A |

<Evaluation of Stability of Ampoule Preparations and Pre-filled Syringe>

Dispersion liquid 1-10 was stored at 40°C for 7 days and examined for $PGE_1$ retention in the same manner as in Example 1-19, except that the container was replaced with the containers shown in Table 1-4. The results obtained are shown in Table 1-4.
[Table 4]

Table 1-4

| | Container | | | $PGE_1$ retention, % |
|---|---|---|---|---|
| | Type | Product | Manufacturer | 40°C, 7 days |
| Example 1-25 | pre-filled syringe | CZ syringe | Daikyo Seikyo | 93% |
| Example 1-26 | ampoule | ordinary ampoule | Fuji Glass | 93% |
| Example 1-27 | ampoule | silicoated ampoule | Fuji Glass | 93% |
| Example 1-28 | ampoule | ordinary ampoule (ampoule white, 1 mL, OP-B) | Namicos | 93% |
| Example 1-29 | ampoule | silicone-treated ampoule (ampoule white, 1 mL, OP-B) | Namicos | 92% |

<Sterilization of Dispersion Liquid>

A 10-mL portion was taken out from dispersion liquid 1-10 and introduced into a silicoated vial (CS-10, manufactured by Fuji Glass Co., Ltd.), and this vial was fitted with a rubber plug and sealed with aluminum. Using an autoclave (Autoclave SP200; Yamato Scientific Co., Ltd.), this was subjected to high-pressure steam sterilization under such conditions that the vial was held at 121°C for 1 minute. The appearance of this liquid was examined and, as a result, separation of oil droplets was observed.

Five hundred milliliters of dispersion liquid 1-10 was subjected to sterilization by filtration in which Sartopore 2 (diameter, 47 mm; pore diameter, 0.2 $\mu$m; Sartorius Stedim Japan) was used as a filter medium for sterilization and a differential pressure of 0.2 MPa was applied with a pressure filter. The whole dispersion liquid was able to be sterilized by filtration without causing clogging. The sterilized dispersion liquid was examined for appearance, particle diameter, pH, and $PGE_1$ content in the same manners as described above. As a result, no significant changes from the state or values determined before the filtration were observed.

(Example 2-1)

Prostaglandin E1 (Alprostadil, manufactured by Daiichi Fine Chemical Co., Ltd.) was dissolved in ethanol in a concentration of 10 mg/mL. A 42-$\mu$L portion thereof (420 $\mu$g in terms of prostaglandin E1) was mixed with 0.252 g of soybean oil (manufactured by Kaneda Co., Ltd.) and 0.504 g of highly purified yolk lecithin PC-98N (manufactured by Q. P. Corp.). A 2.5% aqueous solution of glycerin separately obtained by mixing concentrated glycerin according to the Japanese Pharmacopoeia (manufactured by Kao Corp.) with purified water was added to that mixture in such an amount as to result in a total amount of 60 mL, and the resultant mixture was stirred. This mixture was treated with a homomixer (15,000 rpm, 12 min) to roughly disperse the ingredients and further treated with a chamber type high-pressure homogenizer to emulsify the mixture. A citric acid/sodium citrate buffer was added to the emulsion so as to result in a final concentration of 0.5 mM to adjust the pH of the emulsion to 5.0. Thus, dispersion liquid 2-1 was produced.

(Examples 2-2 to 2-10, Reference Examples 2-1 and 2-2, and Comparative Examples 2-3 and 2-4)

In accordance with the formulations shown in Table 1, dispersion liquids 2-2 to 2-14 were obtained in the same manner as in Example 2-1. Incidentally, the amounts of hydrochloric acid in Example 2-8 and Comparative Examples 2-3 and 2-4 are the amounts of the acid which was added so as to result in the pH value shown in the table.

(Comparative Example 2-5)

The same procedure as in Example 2-1 was conducted, except that the amounts of the soybean oil and highly purified yolk lecithin were changed as shown in Table 2-1, that oleic acid was added and emulsified in an amount of 0.24% based on the dispersion liquid, and that the pH was adjusted to 5.3 using sodium hydroxide in place of the citric acid/sodium citrate buffer. Thus, dispersion liquid 2-15 was obtained. Incidentally, dispersion liquid 2-15 is a dispersion liquid which corresponds to an existent medicine.

(Evaluation of the Fat Emulsions)

<Particle Diameter Measurement>

The dispersion liquids (fat emulsions) prepared in the Examples and Comparative Examples shown in Table 2-1 were each diluted 10-100 times with purified water immediately after the emulsification. The median diameter of a scattered intensity distribution obtained by the CONTIN method with a light-scattering particle size distribution analyzer (FPAR-1000, manufactured by Otsuka Electronics Co., Ltd.) was recorded as the particle diameter.

<pH>

The dispersion liquids prepared in the Examples and Comparative Examples shown in Table 2-1 were each examined, in the undiluted state, for pH with a compact pH meter (manufactured by HORIBA). The measured value was recorded as the pH.

<Storability Test>

A 2-mL portion was taken out from each of the dispersion liquids prepared in the Examples and Comparative Examples shown in Table 2-1, and introduced into a silicoated vial (CS-10, manufactured by Fuji Glass Co., Ltd.). This vial was fitted with a rubber plug and sealed with aluminum. These dispersion liquids were stored at 40°C for 7 days or for 14 days and then subjected to the following tests.

<Evaluation of PGE1 Retention>

The dispersion liquids which were in the state of having been just prepared and the dispersion liquids which had undergone 40°C storage for 7 days or for 14 days were examined for the amount of prostaglandin E1 by high-performance liquid chromatography. In this examination for quantitative analysis, 1-naphthol was used as an internal reference. The PGE1 retention was calculated using the following equation.

$$\text{PGE1 retention (\%)} = [(\text{PGE1 concentration after lapse of time})/(\text{initial PGE1 concentration})] \times 100$$

<Determination of Increase in Content of Free Fatty Acid>

A 1-mL portion of a dispersion liquid is taken out and introduced into a 20-mL vial. Thereto is added 5 mL of a liquid mixture of 2-propanol/heptane/0.5-M sulfuric acid = 40/10/1 (by volume). The liquids are stirred and mixed together. At 10 minutes thereafter, 3 mL of heptane and 3 mL of purified water are added thereto and the ingredients are mixed together by reversing. After the resultant mixture is allowed to stand for 15 minutes, 3 mL of the upper-layer liquid is taken out and introduced into a 10-mL vial. Thereto is added 1 mL of a liquid mixture of 0.02% by mass aqueous Nile Blue solution/ethanol = 1/9 (by volume). The liquids are stirred and mixed together. The resultant liquid mixture is titrated with 0.02-M sodium hydroxide, and the amount of a free fatty acid is calculated using the following equation. This fatty acid amount is converted to the concentration of oleic acid to calculate the content in % by mass based on the whole fat emulsion. The difference in the concentration (% by mass) between the dispersion liquid which had not undergone 40°C storage for 14 days and the dispersion liquid which had undergone the storage is shown in Table 2-1.
Incidentally, a 15 mmol/L heptane solution of oleic acid was used as a reference solution, and V represents the amount titrated.

$$\text{Amount of free fatty acid (meq/L)} = [\text{V (sample)}]/[\text{V (reference solution)}] \times 15$$

The dispersion liquids of Examples 2-1 to 2-10, Reference Examples 2-1 and 2-2, and Comparative Examples 2-3 and 2-4 which had not undergone storage each had a free fatty acid content below the detection limit (n.d.), while the dispersion liquid of Comparative Example 2-5 which had not undergone storage had a free fatty acid content that corresponded to the amount of the oleic acid which had been added.
[Table 5]

Table 2-1

| | | Content in fat emulsion, % | | Component compositional ratio | | Acid | | Initial properties | | Evaluation of stability | | |
|---|---|---|---|---|---|---|---|---|---|---|---|---|
| | | A) Lecithin | B) Oil ingredient | Lecithin/ PGE$_1$ (ratio by mass) | Lecithin/ oil (ratio by mass) | | Amount mM | pH | Particle diameter nm | PGE$_1$ retention, % | | Increase in content of free fatty acid |
| | | PC-98N | Soybean oil | | | | | | | 40°C, 7 days | 40°C, 14 days | |
| Example 2-1 | dispersion liquid 2-1 | 0.84 | 0.42 | 1200 | 2.0 | citric acid | 0.5 | 5.0 | 112 | 92% | 85% | 0.0072% |
| Example 2-2 | dispersion liquid 2-2 | 0.84 | 0.84 | 1200 | 1.0 | citric acid | 0.5 | 5.0 | 114 | 92% | 85% | n.d. |
| Example 2-3 | dispersion liquid 2-3 | 0.84 | 1.68 | 1200 | 0.5 | citric acid | 0.5 | 5.0 | 117 | 93% | 85% | 0.0071% |
| Example 2-4 | dispersion liquid 2-4 | 0.42 | 0.21 | 600 | 2.0 | citric acid | 0.5 | 5.0 | 101 | 91% | 84% | n.d. |
| Example 2-5 | dispersion liquid 2-5 | 1.8 | 0.225 | 2571 | 8.0 | citric acid | 0.5 | 5.0 | 82 | 93% | 85% | 0.0175% |
| Example 2-6 | dispersion liquid 2-6 | 1.8 | 0.45 | 2571 | 4.0 | citric acid | 0.5 | 5.0 | 99 | 93% | 86% | 0.0106% |
| Example 2-7 | dispersion liquid 2-7 | 1.8 | 1.8 | 2571 | 1.0 | citric acid | 0.5 | 5.0 | 93 | 94% | 87% | 0.0142% |
| Example 2-8 | dispersion liquid 2-8 | 0.84 | 0.42 | 1200 | 2.0 | hydrochloric acid | appropriate amount | 5.0 | 64 | 91% | 80% | 0.0160% |
| Example 2-9 | dispersion liquid 2-9 | 1.8 | 3.6 | 2903 | 0.5 | citric acid | 1.5 | 5.3 | 89 | 92% | 88% | 0.0077% |
| Example 2-10 | dispersion liquid 2-10 | 1.8 | 4.0 | 2903 | 0.45 | citric acid | 1.5 | 5.3 | 93 | 93% | 87% | 0.0077% |
| Reference Example 2-1 | dispersion liquid 2-11 | 0.18 | 0.1 | 257 | 1.8 | citric acid | 0.5 | 5.0 | 94 | 90% | 80% | n.d. |
| Reference Example 2-2 | dispersion liquid 2-12 | 1.8 | 0.1 | 2571 | 18.0 | citric acid | 0.5 | 5.0 | 59 | 92% | 78% | 0.0362% |

(continued)

| | | Content in fat emulsion, % | | Component compositional ratio | | Acid | | Initial properties | | Evaluation of stability | | |
|---|---|---|---|---|---|---|---|---|---|---|---|---|
| | | A) Lecithin | B) Oil ingredient | Lecithin/ PGE$_1$ (ratio by mass) | Lecithin/ oil (ratio by mass) | | Amount mM | pH | Particle diameter nm | PGE$_1$ retention, % | | Increase in content of free fatty acid |
| | | PC-98N | Soybean oil | | | | | | | 40°C, 7 days | 40°C, 14 days | |
| Comparative Example 2-3 | dispersion liquid 2-13 | 0.18 | 0.1 | 257 | 18 | hydrochloric acid | appropriate amount | 5.0 | 53 | 90% | 62% | 0.0064% |
| Comparative Example 2-4 | dispersion liquid 2-14 | 1.8 | 0.1 | 2571 | 18.0 | hydrochloric acid | appropriate amount | 5.0 | 49 | 83% | 44% | 0.0778% |
| Comparative Example 2-5 | dispersion liquid 2-15 | 1.8 | 10.0 | 2571 | 0.18 | oleic acid | 8.5 | 5.3 | 227 | 74% | 56% | 0.0159% |

(Examples 2-11 and 2-12 and Comparative Example 2-6)

<Determination of Content of Free Prostaglandin>

A 40-mL portion of each of dispersion liquids 2-1 and 2-6 and comparative dispersion liquid 2-11 is introduced into a vial, and 0.2 mL of a 0.1-M citric acid buffer having a pH of 5.0 is added thereto. Dialysis tube Spectra/Por2 (fractional molecular weight, 12-14 K) is hydrated with purified water, and 2.5% by mass aqueous glycerin solution is enclosed in the dialysis tube in an amount of 1 mL per 20 g of the dispersion liquid. This tube is immersed in the dispersion liquid, and stirring is conducted at 100 rpm and room temperature for 24 hours.

The concentrations of prostaglandin E1 in the dispersion liquid before and after dialysis were determined by HPLC, and the concentration of free PGE1 in the aqueous phase was calculated from the change in concentration through the dialysis. The results obtained are shown in Table 2.

<Blood-pressure Lowering Test>

Inactin is administered to a spontaneously hypertensive rat of the age of 12 weeks to anesthetize the rat. After anesthetized, the rat is held on a warm plate and a cannula is inserted into a femoral artery thereof. The portion is sutured with a yarn. The femoral-artery cannula is connected to a pressure transducer, and average blood pressure for every 5 seconds is begun to be continuously monitored.

After the blood pressure has become stable, each of dispersion liquids 2-1 and 2-6 and comparative dispersion liquid 2-11 was administered through the femoral-artery cannula and the blood pressure is examined for change. The maximum amount by which the blood pressure measured before the administration decreased after the administration of each dispersion liquid was measured. The results obtained are shown in Table 2.

[Table 6]

Table 2-2

| | | Free $PGE_1$ % | Blood pressure before administration mmHg | Decrease in blood pressure mmHg |
|---|---|---|---|---|
| Example 2-11 | dispersion liquid 2-1 | 6% | $153.6 \pm 21.5$ | $45.5 \pm 15.3$ |
| Example 2-12 | dispersion liquid 2-6 | 3% | $163.2 \pm 11.4$ | $45.3 \pm 10.4$ |
| Comparative Example 2-6 | dispersion liquid 2-11 | 20% | $159.2 \pm 13.8$ | $41.1 \pm 17.9$ |

As the results given in Table 2-1 show, it is apparent that dispersion liquids 2-12 and 2-14, in which the mass ratio of the phospholipid to the oil ingredient (A/B in Table 2-1) is larger than 10, come to have a reduced prostaglandin E1 retention after 40°C storage for 2 weeks. These dispersion liquids have a problem that the amount of a free fatty acid which generates with the lapse of time is large; it is hence thought that the lecithin which is present in excess has undergone hydrolysis. It is therefore thought that as the period of the storage stability test becomes longer, not only the stability of the prostaglandin but also the dispersion stability, in terms of emulsified state, of the fat emulsion decreases.

As the results given in Table 2 show, dispersion liquid 2-11, in which the lecithin content is less than 0.4% by mass, contains a large amount of prostaglandin E1 that is present in a free state in the phase. Dispersion liquid 2-11 was less effective in lowering blood pressure and showed a reduced medicinal effect. This is thought to be because a fat emulsion which contains prostaglandin E1 shows a high medicinal effect only when the prostaglandin is present in the oil particles as the dispersoid.

On the other hand, the fat emulsions according to the invention (dispersion liquids 2-1 to 2-10) gave results in which the content of free prostaglandin was low and the prostaglandin retention after storage was high. These results mean that the stability of these fat emulsions is 3 times or more the stability of dispersion liquid 2-15, which corresponds to an existent medicine, and the fat emulsions are expected to attain a considerable prolongation of shelf life. Furthermore, it is apparent that the fat emulsions according to the invention have high emulsion stability and are reduced in the amount of a free fatty acid yielded. In addition, dispersion liquids 2-1 to 2-10 each are a dispersion liquid having a particle diameter of 150 nm or less and are translucent. Consequently, it is easy to detect inclusion of foreign matter in the dispersion liquids.

<Visual Evaluation of Diluted Liquids>

Dispersion liquids 2-1, 2-7, 2-9, and 2-10 were each sampled in an appropriate amount and placed in a 5-mL vial (colorless and transparent), and were diluted 1-6 times with purified water. Each vial was placed between a fluorescent lamp and the naked eye located at a distance of 1 m or more from the lamp, and the diluted liquid was visually examined from the lateral side of the vial. In this examination, the diluted liquid was evaluated in accordance with the following criteria with respect to the degree in which visually recognizable particles were observed in the liquid. The results obtained are shown in Table 2-3.

D: A sediment is clearly seen.
C: Fine particles are seen in small amount.
B: Fine particles are seen only slightly.
A: There are no visually recognizable particles.

[Table 7]

Table 2-3

| | | Content in fat emulsion, % | | Component compositional ratio | | Acid | | Initial properties | | Results of visual evaluation for coarse particles |
|---|---|---|---|---|---|---|---|---|---|---|
| | | A) Lecithin | B) Oil ingredient | Lecithin/ PGE$_1$ (ratio by mass) | Lecithin/ oil (ratio by mass) | | Amount mM | pH | Particle diameter nm | |
| | | PC-98N | Soybean oil | | | | | | | |
| Example 2-13 | dispersion liquid 2-1 | 0.84 | 0.42 | 1200 | 2.0 | citric acid | 0.5 | 5.0 | 112 | C |
| Example 2-14 | dispersion liquid 2-7 | 1.8 | 1.8 | 2571 | 1.0 | citric acid | 0.5 | 5.0 | 93 | B |
| Example 2-15 | dispersion liquid 2-9 | 1.8 | 3.6 | 2903 | 0.5 | citric acid | 1.5 | 5.3 | 89 | A |
| Example 2-16 | dispersion liquid 2-10 | 1.8 | 4.0 | 2903 | 0.45 | citric acid | 1.5 | 5.3 | 93 | A |

<Evaluation of Stability in Ampoule Preparations and Pre-filled Syringe Preparation> Examples 2-17 to 2-21

Dispersion liquid 2-9 was stored at 40°C for 7 days and examined for PGE1 retention in the same manner as in Example 2-9, except that the container to be filled was replaced with the containers shown in Table 2-4. The results obtained are shown in Table 2-4.

[Table 8]

Table 2-4

| | Container | | | PGE$_1$ retention, % |
|---|---|---|---|---|
| | Type | Product | Manufacturer | 40°C, 7 days |
| Example 2-17 | Pre-filled syringe | CZ syringe | Daikyo Seikyo | 93% |
| Example 2-18 | ampoule | ordinary ampoule | Fuji Glass | 93% |
| Example 2-19 | ampoule | silicoated ampoule | Fuji Glass | 93% |
| Example 2-20 | ampoule | ordinary ampoule (ampoule white, 1 mL, OP-B) | Namicos | 93% |
| Example 2-21 | ampoule | silicone-treated ampoule (ampoule white, 1 mL, OP-B) | Namicos | 92% |

<Sterilization of Dispersion liquid>

A 10-mL portion was taken out from dispersion liquid 2-9 and introduced into a silicoated vial (CS-10, manufactured by Fuji Glass Co., Ltd.), and this vial was fitted with a rubber plug and sealed with aluminum. Using an autoclave (Autoclave SP200; Yamato Scientific Co., Ltd.), this was subjected to high-pressure steam sterilization under such conditions that the vial was held at 121°C for 1 minute. The appearance of this liquid was examined and, as a result, separation of oil droplets was observed.

Five hundred milliliters of dispersion liquid 2-9 was subjected to sterilization by filtration in which Sartopore 2 (diameter, 47 mm; pore diameter, 0.2 $\mu$m; Sartorius Stedim Japan) was used as a filter medium for sterilization and a differential pressure of 0.2 MPa was applied with a pressure filter. The whole dispersion liquid was able to be sterilized by filtration without causing clogging. The sterilized dispersion liquid was examined for appearance, particle diameter, pH, and PGE1 content in the same manners as described above.

As a result, no significant changes from the state or values determined before the filtration were observed.

Industrial Applicability

Namely, according to the configurations of the invention, it is possible to provide a prostaglandin-containing fat emulsion, an injection preparation, and a pre-filled syringe preparation which are capable of administration through intravenous injection and have a greatly improved shelf life as compared with conventional products. Furthermore, since this fat emulsion has improved transparency, inclusion of foreign matter can be easily detected and the fat emulsion can be a preparation which is effective also in drug management in clinical fields. Moreover, it is possible to provide a prostaglandin-containing fat emulsion with which a high medicinal effect is obtained.

While the invention has been described in detail and with reference to specific embodiments thereof, it will be apparent to one skilled in the art that various changes and modifications can be made therein without departing from the spirit and scope thereof.

This application is based on a Japanese patent application filed on March 31, 2011 (Application No. 2011-080876), a Japanese patent application filed on March 31, 2011 (Application No. 2011-080877), a Japanese patent application filed on September 6, 2011 (Application No. 2011-194203), and a Japanese patent application filed on September 6, 2011 (Application No. 2011-194204), the contents thereof being incorporated herein by reference.

**Claims**

1. A prostaglandin-containing fat emulsion which is a fat emulsion comprising:

   a prostaglandin compound,

an oil ingredient,
a lecithin,
a water-soluble acid having a pKa of 4.0 to 6.0 and having a dissociable group or a salt thereof, and
water,
wherein the content of the lecithin is 0.15 times or more by mass the content of the oil ingredient, and
the fat emulsion has a pH of 4.5 to 6.0.

2. The prostaglandin-containing fat emulsion according to claim 1,
wherein the water-soluble acid or salt thereof is contained in an amount of 0.01 mmol/L to 5 mmol/L.

3. The prostaglandin-containing fat emulsion according to claim 1 or 2,
wherein the water-soluble acid is citric acid.

4. The prostaglandin-containing fat emulsion according to any one of claims 1 to 3,
wherein the content of the lecithin in the fat emulsion is 0.3 times or more by mass the content of the oil ingredient.

5. The prostaglandin-containing fat emulsion according to any one of claims 1 to 4,
wherein the content of the oil ingredient in the fat emulsion is 0.01 to 5 % by mass based on the fat emulsion.

6. The prostaglandin-containing fat emulsion according to any one of claims 1 to 5, which further contains a higher fatty acid,
wherein the content of the higher fatty acid in the fat emulsion is 0.06 times or less by mass the content of the lecithin.

7. The prostaglandin-containing fat emulsion according to any one of claims 1 to 6, wherein the prostaglandin compound is prostaglandin E1.

8. A prostaglandin-containing fat emulsion which is a fat emulsion comprising:

a prostaglandin compound,
an oil ingredient,
a lecithin, and
water,
wherein the content of the lecithin is 500 to 5,000 times by mass the content of the prostaglandin compound,
the content of the lecithin is 0.3 to 10 times by mass the content of the oil ingredient, and
the content of a higher fatty acid is 0.06 times or less by mass the content of the lecithin.

9. The prostaglandin-containing fat emulsion according to claim 8,
wherein the content of the lecithin is 0.4 to 2% by mass based on the whole fat emulsion.

10. The prostaglandin-containing fat emulsion according to claim 8 or 9,
wherein the portion of the prostaglandin compound which is present in a free state in the aqueous phase accounts for 10% or less of the prostaglandin compound in the fat emulsion.

11. The prostaglandin-containing fat emulsion according to any one of claims 8 to 10,
wherein the content of the oil ingredient is 0.2 to 5% by mass based on the whole fat emulsion.

12. The prostaglandin-containing fat emulsion according to any one of claims 8 to 11,
which further contains a water-soluble acid having a pKa of 4 to 6 and having a dissociable group or a salt thereof.

13. The prostaglandin-containing fat emulsion according to claim 12,
wherein the content of the water-soluble acid or salt thereof in the fat emulsion is 0.01 mmol/L to 5 mmol/L.

14. The prostaglandin-containing fat emulsion according to any one of claims 8 to 13,
wherein the fat emulsion has a pH in the range of 4.5 to 6.0.

15. The prostaglandin-containing fat emulsion according to any one of claims 1 to 14,
wherein an average particle diameter of the fat emulsion as measured by a light scattering method is 30 to 150 nm.

16. The prostaglandin-containing fat emulsion according to any one of claims 1 to 15,
wherein the lecithin is yolk lecithin containing phosphatidylcholine in an amount of 98% by mass or more.

17. The prostaglandin-containing fat emulsion according to any one of claims 1 to 16,
wherein the oil ingredient is soybean oil.

18. The prostaglandin-containing fat emulsion according to any one of claims 1 to 17,
wherein the prostaglandin-containing fat emulsion is a prostaglandin-containing fat emulsion which has undergone sterilization by filtration.

19. An injection preparation which comprises the prostaglandin-containing fat emulsion according to any one of claims 1 to 18.

20. A pre-filled syringe preparation comprising a syringe packed with the prostaglandin-containing fat emulsion according to any one of claims 1 to 18 or with the injection preparation according to claim 19.

21. A process for producing the injection preparation according to claim 19 or 20, which contains a step of sterilizing the prostaglandin-containing fat emulsion according to claim 19 or 20 by filtration.

<table>
<tr><td colspan="2">**INTERNATIONAL SEARCH REPORT**</td><td>International application No.<br>PCT/JP2012/058185</td></tr>
</table>

A. CLASSIFICATION OF SUBJECT MATTER
*A61K47/24*(2006.01)i, *A61K9/107*(2006.01)i, *A61K31/5575*(2006.01)i,
*A61K47/12*(2006.01)i, *A61K47/44*(2006.01)i

According to International Patent Classification (IPC) or to both national classification and IPC

B. FIELDS SEARCHED

Minimum documentation searched (classification system followed by classification symbols)
A61K47/24, A61K9/107, A61K31/5575, A61K47/12, A61K47/44

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CAPLUS/MEDLINE/EMBASE/BIOSIS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X<br>Y | CN 101496787 A (Li, Shubin),<br>05 August 2009 (05.08.2009),<br>entire text; particularly, example 2<br>(Family: none) | 1-3,6,7<br>1-21 |
| Y | WO 2006/118329 A1 (Takeda Chemical Industries, Ltd.),<br>09 November 2006 (09.11.2006),<br>entire text<br>& US 2010/0016381 A1    & EP 1875926 A1<br>& EP 2255785 A2 | 1-21 |
| Y | JP 3-83925 A (Hisamitsu Pharmaceutical Co., Inc.),<br>09 April 1991 (09.04.1991),<br>entire text<br>(Family: none) | 1-21 |

☒ Further documents are listed in the continuation of Box C. ☐ See patent family annex.

| | |
|---|---|
| *   Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search<br>25 May, 2012 (25.05.12) | Date of mailing of the international search report<br>05 June, 2012 (05.06.12) |
|---|---|
| Name and mailing address of the ISA/<br>   Japanese Patent Office<br><br>Facsimile No. | Authorized officer<br><br><br>Telephone No. |

Form PCT/ISA/210 (second sheet) (July 2009)

Form PCT/ISA/210 (continuation of second sheet) (July 2009)

| | INTERNATIONAL SEARCH REPORT | International application No. |
|---|---|---|
| | | PCT/JP2012/058185 |

C (Continuation).　DOCUMENTS CONSIDERED TO BE RELEVANT

| Category* | Citation of document, with indication, where appropriate, of the relevant passages | Relevant to claim No. |
|---|---|---|
| Y | JP 8-81360 A　(Wakamoto Pharmaceutical Co., Ltd.),<br>26 March 1996 (26.03.1996),<br>entire text<br>& US 5693337 A　　　　　& EP 700678 A1 | 1-21 |
| X<br>Y | WO 2009/093650 A1　(Techno Guard Co., Ltd.),<br>30 July 2009 (30.07.2009),<br>entire text<br>(Family: none) | 8,10,14-21<br>1-21 |
| Y | JP 2-203 A　(Nippon Shinyaku Co., Ltd.),<br>05 January 1990 (05.01.1990),<br>entire text<br>& US 5651991 A　　　　　& EP 315079 A1 | 1-21 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8018989 B
- JP 4338333 A
- WO 200993650 A
- JP 8081360 A

- JP 2011080876 A
- JP 2011080877 A
- JP 2011194203 A
- JP 2011194204 A